# EUROPÄISCHE PATENTSCHRIFT

(11) **EP 2 308 560 B1**
(45) Veröffentlichungstag und Bekanntmachung des Hinweises auf die Patenterteilung: **23.11.2011**
(21) Anmeldenummer: 10186656.4
(22) Anmeldetag: 06.10.2010
(51) Int. Cl.: A61N 5/06, H05B 37/02

(54) **Lichttherapievorrichtung**
Light therapy device
Dispositif de luminothérapie

(30) Priorität: 08.10.2009 DE 202009013594 U
(43) Veröffentlichungstag der Anmeldung: 13.04.2011
(73) Patentinhaber: Pässler, Michael, 59174 Kamen (DE)
(72) Erfinder: Pässler, Michael, 59174 Kamen (DE)
(74) Vertreter: Nunnenkamp, Jörg

(56) Entgegenhaltungen:
- EP-A1- 0 320 080
- WO-A1-2009/109387
- WO-A2-2005/059679
- DE-A1- 10 346 076
- US-A- 5 599 274

## Beschreibung

Die Erfindung betrifft eine Lichttherapievorrichtung, mit einem Lichtgehäuse, und mit einer Mehrzahl von LEDs im Lichtgehäuse, die Licht durch eine Öffnung im Lichtgehäuse emittieren, wobei die Helligkeit der LEDs mittels eines pulsweitenmodulierten Betriebsstromes gesteuert wird.

Bei der Lichttherapie handelt es sich um ein von der wissenschaftlichen Medizin anerkanntes Verfahren zur Behandlung verschiedener Erkrankungen mit künstlichem Licht. Mit Lichttherapie werden beispielsweise Depressionen und damit häufig verbundene Schlafstörungen behandelt, welche für betroffene Personen einen Stressfaktor darstellen oder darstellen können. Im Rahmen der Erfindung sei unter dem Begriff Lichttherapie auch die sogenannte Phototherapie verstanden, welche die Behandlung mit Licht bei schweren Beeinträchtigungen der Haut, wie beispielsweise Neurodermitis, bezeichnet. Die Lichttherapie bzw. Phototherapie geht von der Erkenntnis aus, dass insgesamt ohne Sonnenlicht der Mensch krank wird.

Zu diesem Zweck werden Patienten in der Regel einem hellen Kunstlicht ausgesetzt, welches auch als Lichtdusche bezeichnet wird. In diesem Zusammenhang ist nachgewiesen worden, dass Licht den Melatoninhaushaft hemmend beeinflusst, so dass es zu einem positiven Stimmungsumschwung kommt. Tatsächlich wird Melatonin mit depressiven Wirkungen in Verbindung gebracht. Der Stand der Technik nach der DE 601 17 006 T2 befasst sich mit einer transportablen und leichtgewichtigen Augenlichttherapievorrichtung. Diese verfügt in einem zugehörigen Gehäuse über eine Licht emittierende Anordnung einer Mehrzahl von LEDs. Dadurch soll insbesondere Geschäftsreisenden, Schichtarbeitern und Menschen die gefordert sind, weit im geografischen Norden oder Süden zu leben, eine tragbare effektive und wirksame sowie ästhetisch ansprechende Vorrichtung zur Verfügung gestellt werden. In diesem Zusammenhang nutzt die bekannte Vorrichtung Licht emittierende Dioden (LEDs) als Lichtquelle, weil diese leichtgewichtig, klein in der Größe, einfach aufgebaut, dauerhaft und schließlich auch energieeffizient arbeiten.

In diesem Zusammenhang sind die LEDs auf einer Fläche angeordnet und wird im Übrigen ein Diffusorblatt aus einem diffundierenden Blattmaterial über den LEDs positioniert, um eine gleichmäßige Lichtemission zur Verfügung zu stellen. Als LEDs können sowohl Weißlicht-LEDs als auch LEDs verschiedenster Wellenlängen zum Einsatz kommen. Das hat sich grundsätzlich bewährt.

Daneben kennt der Stand der Technik eine Lichttherapievorrichtung, wie sie beispielsweise in der DE 10 2007 033 292 A1 vorgestellt wird. Hier ist eine Lichtkabine mit einer als Leuchte ausgebildeten Kabinenwand realisiert, welche das abgegebene Licht auf ein Kabinenzentrum richtet. In dem Kabinenzentrum ist ein Therapieplatz vorgesehen.

Durch die WO 2005/059679 A2 ist eine gattungsgemäße Lichttherapievorrichtung bekannt geworden, die mit einem Steuerschaltkreis ausgerüstet ist, um einzelne oder eine Vielzahl ausgewählter Signale zu erzeugen. Dazu ist eine mit mehreren LEDs ausgerüstete diffuse Lichtquelle mit einem transluzenten Element realisiert.

Der Stand der Technik kann nicht in allen Aspekten zufriedenstellen. So wird im Rahmen der DE 601 17 006 T2 offensichtlich mit einer mehr oder minder konstanten Beleuchtung respektive Lichtstärke gearbeitet. Das gilt auch für die Lehre nach der DE 10 2007 033 292 A1, welche zudem aufgrund ihrer ausladenden Bauweise kostenaufwendig gestaltet ist und sich kaum für beispielsweise therapeutische Anwendungen in wechselnden Praxisräumen oder auch zu Hause eignet. Hier will die Erfindung insgesamt Abhilfe schaffen.

Der Erfindung liegt das technische Problem zugrunde, eine derartige Lichttherapievorrichtung so weiterzuentwickeln, dass die zusätzliche Möglichkeit der Helligkeitsveränderung besteht, und zwar bei einfachem und kostengünstigen Aufbau. Außerdem geht es darum, unterschiedliche Modi oder Betriebsmodi der LEDs einstellen zu können, um gezielt bestimmte Krankheitsbilder behandeln zu können.

Zur Lösung dieser technischen Problemstellung ist eine gattungsgemäße Lichttherapievorrichtung im Rahmen der Erfindung dadurch gekennzeichnet, dass eine Steuereinheit mit einer Aufnahmeeinrichtung, beispielsweise einem Scanner, zur Erfassung und Nachbildung einer vorgegebenen Farbe vorgesehen ist.

Die Erfindung geht zunächst einmal von der Erkenntnis aus, dass die einzelnen LEDs mit einem konstanten Betriebsstrom beaufschlagt werden, bei welchem es sich vorteilhaft um einen stabilisierten Gleichstrom handelt. Auf diese Weise wird ein sicherer Betrieb der einzelnen LEDs zur Verfügung gestellt und insbesondere gewährleistet, dass sich die jeweilige LED über einen sehr weiten Spannungsbereich mit einem konstanten Strom betreiben lässt. Dieser konstante Betriebsstrom wird nun mit hoher Schaltfrequenz an die jeweils zu beaufschlagende LED angelegt. Die hohe Schaltfrequenz und die Konstanthaltung des Stroms sorgen in diesem Zusammenhang dafür, dass die so betriebene LED für das menschliche Auge flimmerfrei oder flackerfrei leuchtet. Tatsächlich wird hier mit Schaltfrequenzen im Bereich von mehr als 0,1 kHz bis zu ca. 1 kHz gearbeitet.

Außerdem werden die LEDs mittels des pulsweitenmodulierten Betriebsstromes angesteuert. Das heißt, der (gleichgerichtete und konstante) Betriebsstrom zur Beaufschlagung und zum Betrieb der jeweiligen LED erfährt eine Pulsweitenmodulation. Dadurch lässt sich auf elegante Art und Weise die Helligkeit der einzelnen LED einstellen. Die LED kann folglich "gedimmt" werden. Denn im Rahmen der Pulsweitenmodulation wird der (konstante) Betriebsstrom jeweils ein- und ausgeschaltet. Dabei wird zwischen einer Einschaltzeit und eine Ausschaltzeit gewechselt, die jeweils als Rechtecksignale vorliegen. Das Tastverhältnis gibt an, wie lang beispielsweise die Einschaltzeit im Vergleich zur Periodendauer bemessen ist. Beispielsweise korrespondiert ein pulsweitenmoduliertes Signal bzw. PWM-Signal mit einem Tastverhältnis von 25 % dazu, dass über eine Periodendauer gesehen der Strom ein Viertel der Zeit eingeschaltet ist und drei Viertel der Zeit nicht. Je größer das Tastverhältnis umso heller strahlt die auf diese Weise angesteuerte LED.

Die Pulsweitenmodulation wird oftmals durch Oberschwingungen begleitet, welche u. U. als Vielfache der Modulationsfrequenz auftreten. Wenn beispielsweise mit der angesprochenen Schaltfrequenz bzw. Modulationsfrequenz von 0,1 kHz gearbeitet wird, so ist es denkbar, dass zu dieser Frequenz gehörige Oberschwingungen mit 0,2 kHz, 0,3 kHz usw. auftreten. Diese können die ausgestrahlte Lichtintensität der einzelnen LEDs insofern negativ beeinflussen, als beispielsweise ein Flackern, eine Farbänderung etc. beobachtet wird.

Diesen sämtlichen Phänomenen begegnet die Erfindung dadurch, dass der jeweiligen LED nach vorteilhafter Ausgestaltung ein Tiefpass vorgeschaltet ist. Mit Hilfe dieses Tiefpasses werden Signalanteile mit Frequenzen unterhalb einer Grenzfrequenz (beispielsweise 0,1 kHz oder auch 1 kHz) angenähert ungeschwächt passieren gelassen. Das heißt, der Tiefpass stellt sicher, dass die zuvor angesprochenen Oberschwingungen die LED erst gar nicht erreichen und abgeschwächt werden.

Außerdem sorgt der Tiefpass dafür, dass die Rechteckflanken des pulsweitenmodulierten (konstanten) Betriebsstromes hinsichtlich ihrer Steilheit abgerundet oder abgemildert werden. Als Ergebnis wird eine flackerfreie Lichtemission seitens der LEDs zur Verfügung gestellt, welche über den gesamten einstellbaren Helligkeitsbereich zur Verfügung steht. Das ist von besonderer Bedeutung, um die gewünschten therapeutischen Wirkungen zu erzielen und ausdrücklich eine beruhigende Wirkung auf die zu behandelnden Personen auszuüben.

Bei dem eingesetzten Tiefpass handelt es sich im Regelfall um einen Tiefpass erster Ordnung. Das heißt, der Tiefpass greift auf eine Widerstand-Kondensator-Kombination (RC-Glied) zurück. Dabei kann der Tiefpass insgesamt passiv oder aktiv ausgelegt werden. Im erstgenannten Fall ist lediglich das bereits angesprochene RC-Glied realisiert. Im Falle der aktiven Auslegung des Tiefpasses verfügt der Tiefpass zusätzlich noch über ein aktives Bauelement. Hierbei handelt es sich beispielsweise um einen Transistor, welcher insgesamt (in Verbindung mit dem RC-Glied) zur Verbesserung der Filterfunktion des Tiefpasses dient. Dazu ist der Transistor dem RC-Glied nachgeschaltet.

In der Regel handelt es sich bei den LEDs um mehrfarbige LEDs, die beispielsweise als Triplet ausgebildet sein können. Bei einem solchen Triplet werden eine rote, grüne und blaue LED zusammengefasst. Das heißt, die Erfindung greift auf sogenannte RGB-LEDs zurück, welche jeweils einzelne (relativ schmale) Emissionsspektren im sichtbaren Bereich erzeugen. Dabei werden die einzelnen Bestandteile dieses Triplets, also die rote LED, die grüne LED und auch die blaue LED jeweils separat und unabhängig voneinander angesteuert und mit dem pulsweitenmodulierten eigenen Betriebsstrom beaufschlagt. Insgesamt ist die Auslegung so getroffen, dass die sämtlichen im Gehäuse vorhandenen LEDs bzw. RGB-LEDs jeweils parallel zueinander mit dem pulsweitenmodulierten Betriebsstrom beaufschlagt werden, also sämtliche roten LEDs, sämtliche grünen LEDs und sämtliche blauen LEDs gemeinsam. Das geschieht mit Hilfe einer Steuereinheit.

Das heißt, um die einzelnen LEDs einwandfrei und unterschiedlich ansteuern zu können und im Übrigen die Helligkeit mit Hilfe der Pulsweitenmodulation zu variieren, ist zusätzlich die Steuereinheit vorgesehen. Diese Steuereinheit verfügt über wenigstens einen Konstant-Stromausgang, welcher die jeweilige LED mit hoher Schaltfrequenz und unter Berücksichtigung der Pulsweitenmodulation beaufschlagt. Wie bereits erläutert, beträgt die Schaltfrequenz bzw. Modulationsfrequenz des ausgangsseitigen Konstantstromes ca. 0,1 bis 1 kHz. Das ist selbstverständlich nur beispielhaft zu verstehen. Jedenfalls gewährleistet bereits die Untergrenze (0,1 kHz), dass die zugehörige LED vom menschlichen Auge als flimmerfrei oder flackerfrei angesehen wird, weil das menschliche Auge typischerweise Schaltfrequenzen bis allenfalls 30 oder 40 Hz folgen kann.

Im Regelfall weist die Steuereinheit mehrere Konstant-Stromausgänge auf. Für den Fall, dass es sich bei den LEDs um RGB-LEDs bzw. Triplets aus einer roten, grünen und blauen LED handelt, sind drei Konstant-Stromausgänge realisiert. Jeder Konstant-Stromausgang korrespondiert zu einer Farbe (rot, grün, blau) und ist mit den entsprechend gestalteten Einzel-LEDs der RGB-LEDs verbunden. Auf diese Weise können die sämtlichen roten LEDs, die sämtlichen grünen und die sämtlichen blauen LEDs unterschiedlich voneinander ein- und ausgeschaltet werden, in ihrer Helligkeit eine Änderung erfahren etc.. Das alles gibt die Steuereinheit vor.

Dadurch lässt sich die Steuereinheit insgesamt frei programmierbar auslegen. Mit Hilfe der Steuereinheit kann also grundsätzlich jede beliebige Farbe des emittierten Lichtes erzeugt werden, beispielsweise durch additive Farbmischung der drei Grundfarben rot, grün und blau. Darüber hinaus lassen sich unterschiedliche Leuchtdauem der sämtlichen LEDs oder der Einzel-LEDs realisieren. Auch wechselnde Farbreihenfolgen sind denkbar. Ebenso, dass beispielsweise unterschiedliche Farben ineinander übergehen. Ganz abgesehen davon lassen sich die LEDs konstant oder auch im Pulsbetrieb betreiben.

Der Pulsbetrieb korrespondiert dazu, dass sich die einzelnen LEDs - unabhängig von der PWM-Modulation - für bestimmte vorgebbare Zeiten ein- und ausschalten lassen. Dabei korrespondiert der Pulsbetrieb meistens zu Frequenzen von wenigen Hertz, ist also der PWM-Beaufschlagung der einzelnen LEDs mit wenigstens 0,1 kHz praktisch überlagert. Selbstverständlich können mit Hilfe der Steuereinheit auch noch weitere Modi bzw. Betriebsmodi der LEDs realisiert werden.

Darüber hinaus hat es sich bewährt, wenn die Steuereinheit mit einer Fembedieneinrichtung ausgerüstet ist bzw. eine solche Fembedieneinrichtung der Steuereinheit zugeordnet ist. Bei der Fembedieneinrichtung mag es sich um ein Handgerät mit Bedienknöpfen aber auch einen Computer oder allgemein eine Rechnereinheit handeln, mit deren Hilfe sich programmierbare Abläufe hinsichtlich des Farbwechsels, der Farbe, der Ansteuerung der LED usw. vorgeben lassen. Darüber hinaus verfügt die Steuereinheit über einen Scanner als Aufnahmeeinrichtung.

Mit Hilfe der Aufnahmeeinrichtung bzw. des Scanners kann beispielsweise eine vorgegebene Farbe erfasst und nachgebildet werden. Das ist von besonderer Bedeutung vor dem Hintergrund, dass bisherige Lichttherapievorrichtungen oft-mals noch auf vorgeschaltete Filter oder mehrere Filterscheiben zurückgreifen. Soll nun das von einer solchen Filterscheibe erzeugte emittierte Licht mit Hilfe der erfindungsgemäßen Lichttherapievorrichtung nachgeahmt werden, so ist es lediglich erforderlich, die "Farbe" des fraglichen Filters mit Hilfe der Aufnahmeeinrichtung zu erfassen und dann unter Rückgriff auf die LEDs in Verbindung mit der Steuereinheit nachzubilden.

Die fragliche Steuereinheit ist im Regelfall im Lichtgehäuse angeordnet. Demgegenüber findet sich die Fernbedieneinrichtung meistens in einem separaten Fernbediengehäuse. Außerdem kann die Steuereinheit an ein externes Datennetz, beispielsweise das Internet, zur Fernsteuerung angeschlossen werden. Dadurch lassen sich beispielsweise Updates der in der Steuereinheit eingesetzten Software von außen aufspielen. Auch ist es denkbar, die Steuereinheit grundsätzlich von einer externen Zentrale aus fernzubedienen.

Wie bereits eingangs erläutert, verfügt das Lichtgehäuse über eine Öffnung, durch welche das von den LEDs emittiert Licht nach außen austritt. In diese Öffnung ist im Regelfall eine Streuscheibe eingesetzt, bei welcher es sich meistens um eine (weiße) Milchglasscheibe handelt. Jedenfalls verfügt die Streuscheibe über eine Vielzahl an Unregelmäßigkeiten bzw. Streuzentren an ihrer Oberfläche und/oder im Innern, so dass das von den einzelnen LEDs emittierte Licht an der Streuscheibe diffus gestreut wird. Dadurch wird in Verbindung mit dem flackerfreien Betrieb der LEDs insgesamt ein besonders vorteilhafter Lichteffekt im Sinne einer Lichtdurchflutung erreicht. Auch Übergänge einzelner Farben lassen sich "weich" realisieren, weil die Steuereinheit insofern für eine additive Farbmischung der einzelnen LEDs sorgt und ein gleichsam fließender Übergang von der einen zur anderen Farbe realisiert wird.

Um die gesamte Lichttherapievorrichtung transportabel und universell einsetzbar zu gestalten, sind das Lichtgehäuse und das Fernbediengehäuse im Regelfall gemeinsam an eine Ständereinheit angeschlossen. Diese Ständereinheit ist transportabel ausgeführt, lässt sich beispielsweise zerlegen. Darüber hinaus mag die Ständereinheit verfahrbar ausgelegt sein, um unterschiediiche Aufstellungsorte in einem Behandlungszimmer zu realisieren.

Im Ergebnis wird eine Lichttherapieeinheit zur Verfügung gestellt, die durch eine besonders günstige Lichtabstrahlung überzeugt. Tatsächlich wird die Lichtabstrahlung und insbesondere die Variation ihrer Helligkeit über eine Pulsweitenmodulation des konstanten Betriebsstromes erreicht. Da darüber hinaus den jeweiligen LEDs ein Tiefpass vorgeschaltet ist, werden störende Oberschwingungen unterdrückt und fließende Übergänge erzeugt.

In Verbindung mit der Möglichkeit der freiprogrammierbaren Auslegung der Steuereinheit werden ganz neue Therapiefelder erschlossen. Denn es können je nach Vorgabe praktisch beliebige Farben ausgangsseitig erzeugt werden und auch bestimmte Farbprogramme vorgegeben werden. Diese lassen sich je nach Krankheitsbild und Anforderungsprofil der zu behandelnden Person entweder fest vorgeben oder aber auch mit Hilfe der Fernbedieneinrichtung bzw. eines an dieser Stelle eingesetzten Rechners oder Computers selbst programmieren.

Die frei programmierbare Auslegung der Steuereinheit ermöglicht darüber hinaus die Kombination der beschriebenen Lichttherapievorrichtung mit einem computerbasierten Analyse- und Therapiesystem, dessen Ausgangswerte als Eingangswerte für die Steuereinheit genutzt werden. Bei den fraglichen Analyse- und Therapiesystemen kann es sich um ein Radionik-Gerät handeln. Mit dessen Hilfe kann anhand einer Haar- oder Speichel probe eines Patienten dessen sogenanntes Energiefeld ermittelt werden. Mit Hilfe eines Computerprogramms im Radionik-Gerät lässt sich aus den gewonnenen Daten eine Heilungsinformation erhalten, die den gewünschten therapeutischen Effekt erzielen soll. Diese Heilungsinformation korrespondiert zu einem bestimmten Farbprogramm und kann folglich zur Ansteuerung der Steuereinheit der Lichttherapievorrichtung genutzt werden.

Infolge der transportablen und kompakten Auslegung lässt sich die beschriebene Lichttherapievorrichtung nicht nur in speziell ausgerüsteten Praxisräumen anwenden, sondern kann praktisch in beliebigen Räumen zum Einsatz kommen. Hier sind beispielsweise auch Hotels, Kosmetiksalons etc. denkbar. Darin sind die wesentlichen Vorteile zu sehen.

Im Folgenden wird die Erfindung anhand einer lediglich ein Ausführungsbeispiel darstellenden Zeichnung näher erläutert; es zeigen:
- Fig. 1: die erfindungsgemäß Lichttherapievorrichtung perspektivisch,
- Fig. 2: einen Schnitt durch das Lichtgehäuse,
- Fig. 3A: die Steuereinheit schematisch,
- Fig. 3B: ein Ausgangssignal der Steuereinheit und
- Fig. 4: die zur Steuereinheit gehörige Fembedieneinrichtung in einer Über- sicht.

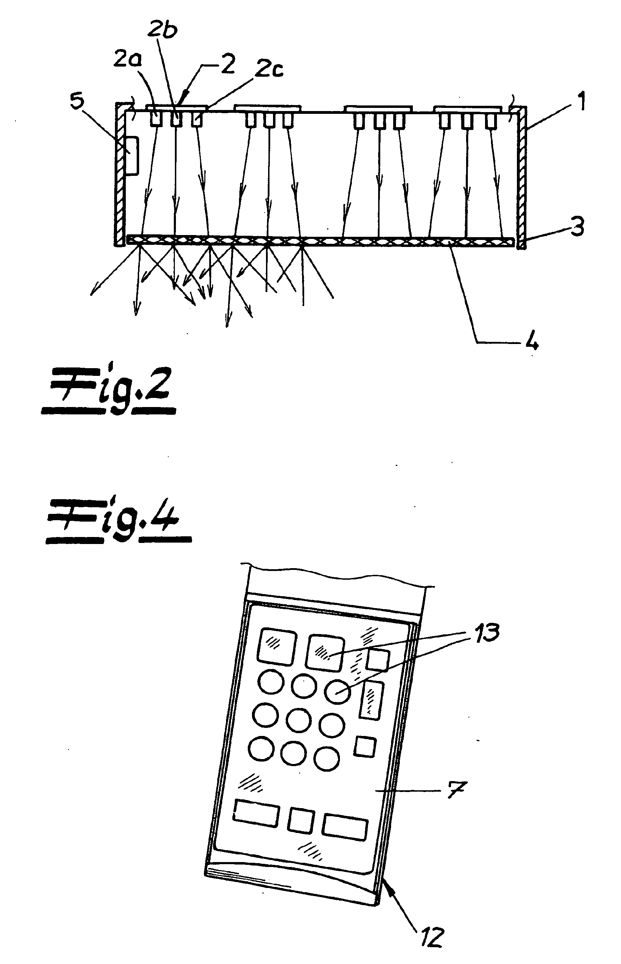

In den Figuren ist eine Lichttherapievorrichtung dargestellt, also eine Vorrichtung, die zur Lichttherapie eingesetzt wird und insbesondere die Seratoninproduktion anspricht. Zu diesem Zweck verfügt die Lichttherapievorrichtung in ihrem grundsätzlichen Aufbau über ein Lichtgehäuse 1 und eine Mehrzahl von LEDs 2 in dem Lichtgehäuse 1. Außerdem ist das Lichtgehäuse 1 mit einer Öffnung 3 ausgerüstet, durch welche die LEDs 2 ihr produziertes Licht nach außen emittieren. Im Rahmen des Ausführungsbeispiels ist die fragliche Öffnung 3 mit einer Streuscheibe 4 verschlossen, bei welcher es sich beispielhaft um eine weiße Milchglasscheibe handelt oder handeln kann. Jedenfalls sorgt die Streuscheibe 4 dafür, dass das von den LEDs 2 ausgehende und emittierte Licht beim Durchtritt durch die Streuscheibe 4 diffus gestreut wird, wie dies in der Fig. 2 schematisch angedeutet ist. Dadurch wird im Bereich der Öffnung 3 ein insgesamt homogener Lichteindruck erzeugt und fungieren die LEDs 2 als gleichsam Lichtduschen zur Lichtflutung.

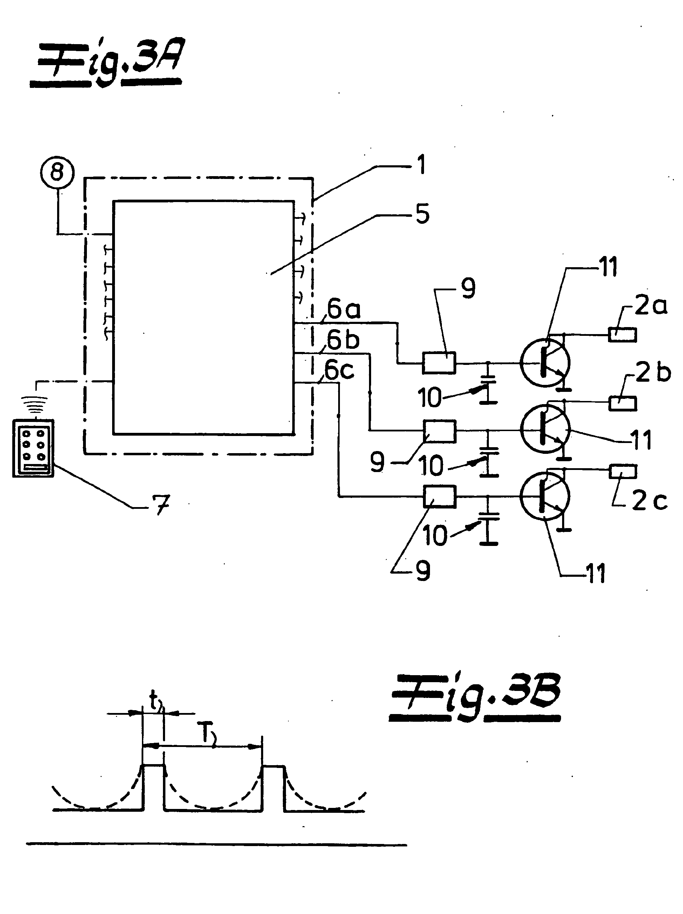

Erfindungsgemäß lässt sich nun die Helligkeit der LEDs 2 auf besondere Art und Weise variieren und steuern. Zu diesem Zweck ist eine Steuereinheit 5 vorgesehen, die schematisch in der Fig. 3A dargestellt ist. Die Steuereinheit 5 findet sich wie die LEDs 2 im Innern des Lichtgehäuses 1. Ausweislich der Fig. 3A ist die Steuereinheit 5 mit drei Konstant-Stromausgängen 6a, 6b, 6c ausgerüstet.

Jeder Konstant-Stromausgang 6a, 6b, 6c dient dazu, eine Gruppe von LEDs 2 mit Gleichspannung zu versorgen, und zwar unter Berücksichtigung eines stabilisierten Gleichstromes. Da es sich bei den LEDs 2 um RGB-LEDs 2 handelt, korrespondiert jeder Konstant-Stromausgang 6a, 6b, 6c zu einer Farbe dieser RGB-LEDs 2. Tatsächlich ist die jeweilige LED 2 als Triplet ausgelegt und verfügt auf diese Weise über einen mehrfarbigen Charakter. Im Rahmen des Ausführungsbeispiels weist jede LED 2 eine rote LED 2a, eine grüne LED 2b sowie schließlich eine blaue LED 2c auf. Diese sind zwar in einem gemeinsamen Gehäuse angeordnet, lassen sich allerdings unterschiedlich und unabhängig voneinander beaufschlagen.

Anhand der Fig. 3A wird deutlich, dass an den einen Konstant-Stromausgang 6a der Steuereinheit 5 sämtliche roten LEDs 2a angeschlossen sind. Der weitere Konstant-Stromausgang 6b dient dagegen zur Stromversorgung der sämtlichen grünen LEDs 2b. Die blauen LEDs 2c sind schließlich an den weiteren dritten Konstant-Stromkreis 6c angeschlossen. Folgerichtig verfügt die Steuereinheit 5 über mehrere, im Ausführungsbeispiel drei, Konstant-Stromausgänge 6a, 6b und 6c, welche den unterschiedlich farbigen LEDs 2a, 2b, 2c zugeordnet sind.

Der Konstant-Stromausgang 6 bzw. die einzelnen Konstant-Stromausgänge 6a, 6b, 6c stellen ausgangsseitig einen jeweils pulsweitenmodulierten Betriebsstrom für die zugehörige LED 2a, 2b, 2c zur Verfügung. Dieser pulsweitenmodulierte Betriebsstrom ist in der Fig. 3B schematisch angedeutet. Wie üblich kann mit Hilfe der Pulsweitenmodulation (PWM) das sogenannte Tastverhältnis t/T bei konstanter Frequenz 1/T moduliert werden. Die Frequenz wird durch die Periodendauer T vorgegeben und ist vorliegend konstant, mag beispielsweise im Bereich zwischen 0,1 kHz bis 1 kHz angesiedelt sein, damit die fragliche LED 2 bzw. die einzelnen LEDs 2a, 2b, 2c für einen Betrachter auf jeden Fall flacker- oder flimmerfrei wirken. Die Schaltfrequenz bzw. Modulationsfrequenz, mit deren Hilfe die einzelnen LEDs 2a, 2b, 2c angesteuert werden, entspricht dem Kehrwert der Periodendauer T, also 1/T.

Das Tastverhältnis t/T gibt nun das Verhältnis einer Einschaltzeit t des (konstanten) Betriebsstromes für die einzelnen LEDs 2a, 2b, 2c im Vergleich zur Periodendauer T vor, also t/T. Dieses Tastverhältnis t/T beträgt im in der Fig. 3B gezeigten Beispielfall ca. 25 %. Indem das Tastverhältnis t/T vergrößert wird, lässt sich die Helligkeit der entsprechend angesteuerten Leuchtdiode 2a, 2b, 2c steigern. Umgekehrt korrespondiert ein verringertes Tastverhältnis t/T zu einer abnehmenden Helligkeit.

Da sämtliche Leuchtdioden 2a, 2b, 2c des Triplets bzw. der jeweiligen RGB-LED 2 unterschiedlich angesteuert werden können, lassen sich die Farben rot, blau und grün mit wechselnder Helligkeit darstellen und können im Übrigen additiv gemischt werden. Dadurch lässt sich prinzipiell jede denkbare Farbe durch additive Farbmischung unter Rückgriff auf die drei Grundfarben rot, grün und blau darstellen. - Selbstverständlich können auch andere Färben oder Pastelltöne Verwendung finden.

Darüber hinaus können die Einzel-LEDs 2a, 2b, 2c wie auch die einzelnen RGB-LEDs 2 mit unterschiedlichen Leuchtdauern seitens der Steuereinheit 5 beaufschlagt werden. Außerdem wird deutlich, dass sich mit Hilfe der Steuereinheit 5 wechselnde Farbreihenfolgen vorgeben lassen. Ganz unabhängig davon ist die Steuereinheit 5 auch in der Lage, die LEDs 2 insgesamt konstant oder im Impulsbetrieb zu beaufschlagen. Dabei korrespondiert der Impulsbetrieb zu einer Frequenz von lediglich einigen Hertz, liegt also mit seiner Frequenz mehrere Größenordnungen unterhalb der Modulationsfrequenz bzw. Schaltfrequenz, wie sie im Rahmen der PWM-Modulation den einzelnen LEDs 2 aufgeprägt wird (0,1 kHz im Beispielfall).

Anhand dieser Erläuterungen wird deutlich, dass die Steuereinheit 5 frei programmierbar ausgebildet ist. Zu diesem Zweck ist der Steuereinheit 5 eine Fernbedieneinrichtung 7 zugeordnet, bei welcher es sich im Rahmen der Fig. 4 um ein externes Bedienpaneel oder ein Handgerät handelt. Grundsätzlich kann die Fembedieneinrichtung 7 auch als Computer 7 oder allgemein Rechner ausgelegt sein, mit dessen Hilfe die gewünschte Farbe, die Leuchtdauern, etwaige Farbreihenfolgen, Ein- und Ausschaltzeiten, Helligkeiten etc. der Einzel-LEDs 2a, 2b, 2c wie auch der LEDs 2 insgesamt vorgegeben und programmiert werden können.

Nicht dargestellt ist die weitere Möglichkeit, die Steuereinheit 5 mit einer Aufnahmeeinrichtung zu flankieren. Bei dieser Aufnahmeeinrichtung mag es sich um einen Scanner handeln, welcher zur Erfassung und Nachbildung einer vorgegebenen Farbe dient. Dieser Scanner mag beispielsweise die Farbe eines bisher eingesetzten Filters abtasten, die anschließend als Eingangssignal für die Steuereinheit 5 genutzt wird. Mit Hilfe der Steuereinheit 5 werden im Anschluss daran die Einzel-LEDs 2a, 2b, 2c im Sinne einer Regelung so beeinflusst bzw. beaufschlagt, dass die zuvor aufgenommene Farbe oder Farbgebung nachgebildet ist.

Anhand der Fig. 3A wird des Weiteren deutlich, dass die Steuereinheit 5 an ein externes Datennetz 8 angeschlossen ist oder angeschlossen werden kann. Über dieses externe Datennetz 8 lassen sich Vorgaben für die Steuereinheit 5 von außen einspeisen oder kann die Steuereinheit 5 sogar von einem entfernten Ort aus bedient und angesteuert werden.

Als weitere Besonderheit ist im Rahmen der Erfindung jeder Einzel-LED 2a, 2b, 2c bzw. der jeweiligen LED 2 ein Tiefpass 9, 10, 11 vorgeschaltet. Bei diesem Tiefpass 9, 10, 11 handelt es sich um einen solchen erster Ordnung, also die Kombination aus einem Widerstand 9 und einem Kondensator 10, also ein sogenanntes RC-Glied 9, 10. Darüber hinaus ist der Tiefpass 9, 10, 11 aktiv ausgelegt, denn er verfügt ergänzend zu dem RC-Glied 9, 10 über ein aktives Bauelement 11. Dieses aktive Bauelement 11 stellt vorliegend einen Transistor 11 dar, welcher dem RC-Glied 9, 10 zur Verbesserung seiner Filterfunktion zugeordnet ist und dem RC-Glied 9, 10 folgt.

Infolge des beschriebenen Tiefpasses 9, 10, 11 werden durch die PWM-Modulation gegebenenfalls eingangsseitig an den Einzel-LEDs 2a, 2b, 2c beobachtete Oberschwingungen unterdrückt. Im Übrigen sorgen die einzelnen Tiefpässe 9, 10, 11 dafür, dass die Rechteckflanken bei der PWM-Modulation abgeschrägt werden, wie dies gestrichelt in der Fig. 3B angedeutet ist. Dadurch kommt es insgesamt zu einem besonders stabilen und flackerfreien Lichteindruck und lassen sich insbesondere auch harmonische und gleitende Übergänge von einer zur anderen Farbe realisieren.

Dabei ist die Auslegung insgesamt so getroffen, dass jeweils ein Tiefpass 9, 10, 11 für jede Farbe (rot, grün, blau) vorgesehen ist, wie die Fig. 3A deutlich macht. Das heißt, die an der Steuereinheit 5 vorgesehenen Konstant-Stromausgänge 6a, 6b, 6c sind jeweils mit einem zugehörigen Tiefpass 9, 10, 11 ausgerüstet. Ausgangsseitig dieses Tiefpasses 9, 10, 11 wird das entsprechend geglättete PWM-Modulationssignal jeweils an die LEDs 2a, 2b, 2c gleicher Farbe weitergeleitet. Hier sorgt das entsprechend tiefpassgefilterte und folglich auch geglättete Signal bzw. der stabilisierte Gleichstrom dafür, dass die zugehörige Einzel-LED 2a, 2b, 2c mit der gewünschten Helligkeit strahlt. Mittels weiterer Einzel-LEDs 2a, 2b, 2c wird dann durch additive Farbmischung der gewünschte Farbeindruck erzeugt. Dieser erfährt durch die diffuse Streuung an der Streuscheibe 4 eine Vergleichmäßigung, so dass im Ergebnis die gesamte Streuscheibe 4 in der gewünschten Farbe erscheint. Denn die LEDs 2 sind gleichmäßig über die Fläche der Öffnung 3 verteilt im Innern des Lichtgehäuses 1 angeordnet.

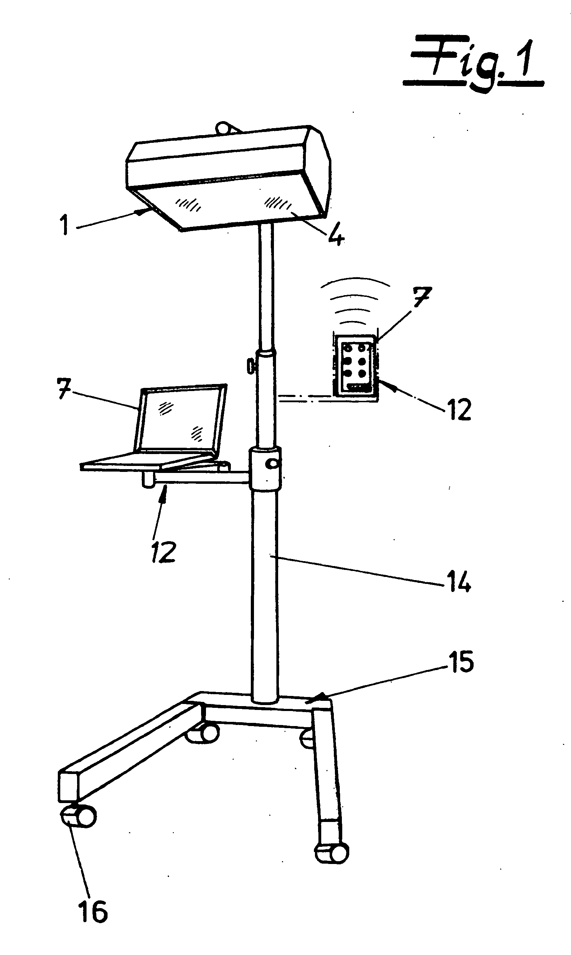

Zusätzlich zu dem Lichtgehäuse 1 ist noch ein Fernbediengehäuse 12 realisiert, welches die Fembedieneinrichtung 7 aufnimmt. Anhand der Fig. 4 erkennt man, dass die Fembedieneinrichtung 7 frontseitig verschiedene Bedienknöpfe 13 aufweist, mit deren Hilfe beispielsweise unterschiedliche und fest eingestellte Farbprogramme, Farben, Helligkeitswerte etc. abgerufen werden können und die Steuereinheit 5 entsprechend beaufschlagen, um die LEDs 2 im gewünschten Sinne anzusteuern. Anhand der Fig. 1 erkennt man, dass sowohl das Lichtgehäuse 1 als auch ein Fernbediengehäuse 12 für die Fernbedieneinrichtung 7 gemeinsam an eine Ständereinheit 14, 15 angeschlossen sind. Die Ständereinheit 14, 15 setzt sich grundsätzl ich aus einem Vertikalständer 14 und einem Fuß 15 zusammen. Insgesamt mag die Ständereinheit 14, 15 transportabel gestaltet sein, um eine flexible Aufstellung zu ermöglichen. In diese Richtung zielen auch verfahrbare Rollen 16 am Fuß 15, die die erforderliche Verfahrbarkeit der Ständereinheit 14, 15 im Ganzen sicherstellen.

## Patentansprüche

1. Lichttherapievorrichtung, mit einem Lichtgehäuse (1), und mit einer Mehrzahl von LEDs (2) im Lichtgehäuse (1), die Licht durch eine Öffnung (3) im Lichtgehäuse (1) emittieren, wobei die Helligkeit der LEDs (2) mittels eines pulsweitenmodulierten Betriebsstromes steuerbar ist, **gekennzeichnet durch** eine Steuereinheit (5) mit einer Aufnahmeeinrichtung zur Erfassung und Nachbildung einer vorgegebenen Farbe.

2. Vorrichtung nach Anspruch 1, **dadurch gekennzeichnet, dass** der jeweiligen LED (2) ein Tiefpass (9, 10, 11) vorgeschaltet ist.

3. Vorrichtung nach Anspruch 2, **dadurch gekennzeichnet, dass** der Tiefpass (9, 10, 11) als Tiefpass erster Ordnung ausgebildet ist.

4. Vorrichtung nach Anspruch 2 oder 3, **dadurch gekennzeichnet, dass** der Tiefpass (9,10,11) ein aktives Bauelement (11), beispielsweise einen Transistor (11), zur Verbesserung seiner Filterfunktion aufweist.

5. Vorrichtung nach einem der Ansprüche 1 bis 4, **dadurch gekennzeichnet, dass** die LEDs (2) mehrfarbig, beispielsweise als Triplet aus einer roten LED (2a), einer grünen LED (2b) und einer blauen LED (2c) ausgebildet sind.

6. Vorrichtung nach einem der Ansprüche 1 bis 5, **dadurch gekennzeichnet, dass** eine Steuereinheit (5) mit wenigstens einem Konstanz-Stromausgang (6a, 6b, 6c) vorgesehen ist, welcher die jeweiligen LEDs (2a, 2b, 2c) mit hoher Schaltfrequenz von beispielsweise 0,1 kHz bis 1 kHz beaufschlagt.

7. Vorrichtung nach Anspruch 6, **dadurch gekennzeichnet, dass** die Steuereinheit (5) mehrere Konstanz-Stromausgänge (6a, 6b, 6c) aufweist, welche jeweils den unterschiedlich farbigen LEDs (2a, 2b, 2c) zugeordnet sind.

8. Vorrichtung nach Anspruch 6 oder 7, **dadurch gekennzeichnet, dass** die Steuereinheit (5) frei programmierbar ausgebildet ist und beispielsweise verschiedene Farben oder Modi des durch die LEDs (2a, 2b, 2c) emittierten Lichtes durch additive Farbmischung, unterschiedliche Leuchtdauem, Farbreihenfolgen, Helligkeiten oder, Konstant-/Impulsbestrahlung vorgibt.

9. Vorrichtung nach einem der Ansprüche 6 bis 8, **dadurch gekennzeichnet, dass** der Steuereinheit (5) eine Fembedieneinrichtung (7) zugeordnet ist, beispielsweise ein Handgerät (7) oder, ein Computer (7) .

10. Vorrichtung nach einem der Ansprüche 1 bis 9, **dadurch gekennzeichnet, dass** die Steuereinheit (5) an ein externes Datennetz (8) zur Fernsteuerung anschlieβbar ist.

11. Vorrichtung nach einem der Ansprüche 1 bis 10, **dadurch gekennzeichnet, dass** in die Öffnung (3) im Lichtgehäuse (1) eine Streuscheibe (4), beispielsweise eine Milchglasscheibe (4), eingesetzt ist.

12. Vorrichtung nach Anspruch 9 **dadurch gekennzeichnet, dass** die Steuereinheit (5) im Lichtgehäuse (1) angeordnet ist, während die Fembedieneinrichtung (7) über ein separates Fernbediengehäuse (12) verfügt.

13. Vorrichtung nach Anspruch 12, **gekennzeichnet durch** eine Ständereinheit (14, 15), an welche das Lichtgehäuse (1) und das Fernbediengehäuse (12) gemeinsam angeschlossen sind.

14. Vorrichtung nach Anspruch 13, **dadurch gekennzeichnet, dass** die Ständereinheit (14) transportabel und/oder verfahrbar ausgebildet ist.

## Claims

1. Light therapy device comprising a light housing (1) and comprising a plurality of LEDs (2) in the light housing (1) which emit light through an opening (3) in the light housing (1), the brightness of the LEDs (2) being able to be controlled by means of a pulse-width modulated operating current, **characterised by** a control unit (5) comprising a recording device for recording and simulating a predetermined colour.

2. Device according to Claim 1, **characterised in that** a low pass filter (9, 10, 11) is arranged upstream of the respective LED (2).

3. Device according to Claim 2, **characterised in that** the low pass filter (9, 10, 11) is configured as a low pass filter of the first order.

4. Device according to Claim 2 or 3, **characterised in that** the low pass filter (9, 10, 11) comprises an active component (11), for example a transistor (11), for improving the filtering function thereof.

5. Device according to one of Claims 1 to 4, **characterised in that** the LEDs (2) are configured to be multicoloured, for example as a triplet consisting of a red LED (2a), a green LED (2b) and a blue LED (2c).

6. Device according to one of Claims 1 to 5, **characterised in that** a control unit (5) is provided, comprising at least one constant current output (6a, 6b, 6c), and which subjects the respective LEDs (2a, 2b, 2c) to a high switching frequency of, for example, 0.1 kHz to 1 kHz.

7. Device according to Claim 6, **characterised in that** the control unit (5) comprises a plurality of constant current outputs (6a, 6b, 6c) which in each case are associated with the different coloured LEDs (2a, 2b, 2c).

8. Device according to Claim 6 or 7, **characterised in that** the control unit (5) is configured to be freely programmable and, for example, predetermines different colours or modes of the light emitted by the LEDs (2a, 2b, 2c) by additive colour mixing, variable illumination duration, colour sequences, brightness or constant/pulsed radiation.

9. Device according to one of Claims 6 to 8, **characterised in that** a remote control device (7) is associated with the control unit (5), for example a handheld device (7) or a computer (7).

10. Device according to one of Claims 1 to 9, **characterised in that** the control unit (5) is able to be connected to an external data network (8) for remote control.

11. Device according to one of Claims 1 to 10, **characterised in that** a diffuser (4), for example a frosted glass panel (4), is inserted into the opening (3) in the light housing (1).

12. Device according to Claim 9, **characterised in that** the control unit (5) is arranged in the light housing (1), whilst the remote control device (7) uses a separate remote control housing (12).

13. Device according to Claim 12, **characterised by** a support unit (14, 15) to which the light housing (1) and the remote control housing (12) are both connected.

14. Device according to Claim 13, **characterised in that** the support unit (14) is configured to be transportable and/or movable.

## Revendications

1. Dispositif de thérapie par la lumière comprenant un boîtier de lumière (1) et une pluralité de LEDs (2) dans le boîtier de lumière (1), qui émettent de la lumière par une ouverture (3) située dans le boîtier de lumière (1), la luminosité des LEDs (2) pouvant être commandée au moyen d'un courant de service modulé au niveau de la largeur d'impulsion, **caractérisé par** une unité de commande (5) dotée d'un dispositif d'enregistrement pour la saisie et la reproduction d'une couleur prédéfinie.

2. Dispositif selon la revendication 1, **caractérisé en ce qu'**un filtre passe-bas (9, 10, 11) est disposé en amont de la LED (2) respective.

3. Dispositif selon la revendication 2, **caractérisé en ce que** le filtre passe-bas (9, 10, 11) est conçu comme filtre passe-bas de premier ordre.

4. Dispositif selon la revendication 2 ou 3, **caractérisé en ce que** le filtre passe-bas (9, 10, 11) présente un composant (11) actif, par exemple un transistor (11), pour l'amélioration de sa fonction de filtre.

5. Dispositif selon l'une quelconque des revendications 1 à 4, **caractérisé en ce que** les LEDs (2) sont conçues en plusieurs couleurs, par exemple sous forme de triplet constitué d'une LED rouge (2a), d'une LED verte (2b) et d'une LED bleue (2c).

6. Dispositif selon l'une quelconque des revendications 1 à 5, **caractérisé en ce qu'**il est prévu une unité de commande (5) dotée d'au moins une sortie de courant à constance (6a, 6b, 6c), laquelle alimente les LEDs (2a, 2b, 2c) respectives avec une fréquence de commutation élevée allant de par exemple 0,1 kHz à 1 kHz.

7. Dispositif selon la revendication 6, **caractérisé en ce que** l'unité de commande (5) présente plusieurs sorties de courant à constance (6a, 6b, 6c), lesquelles sont attribuées respectivement aux LEDs (2a, 2b, 2c) de couleur différente.

8. Dispositif selon la revendication 6 ou 7, **caractérisé en ce que** l'unité de commande (5) est conçue de façon librement programmable et prédéfinit par exemple différentes couleurs ou différents modes de la lumière émise par les LED (2a, 2b, 2c) par mélange additif de couleurs, différentes durées d'éclairage, séquences de couleurs, luminosités ou rayonnement constant/rayonnement par impulsions.

9. Dispositif selon l'une quelconque des revendications 6 à 8, **caractérisé en ce qu'**un dispositif de télécommande (7), par exemple un appareil portatif (7) ou un ordinateur (7), est attribué à l'unité de commande (5).

10. Dispositif selon l'une quelconque des revendications 1 à 9, **caractérisé en ce que** l'unité de commande (5) peut être raccordée à un réseau de données (8) externe pour la télécommande.

11. Dispositif selon l'une quelconque des revendications 1 à 10, **caractérisé en ce qu'**un disque diffuseur (4), par exemple un disque à verre dépoli (4), est inséré dans l'ouverture (3) dans le boîtier de lumière (1).

12. Dispositif selon la revendication 9, **caractérisé en ce que** l'unité de commande (5) est disposée dans le boîtier lumineux (1), alors que le dispositif de télécommande (7) dispose d'un boîtier de télécommande (12) séparé.

13. Dispositif selon la revendication 12, **caractérisé par** une unité de montant (14, 15), sur laquelle le boîtier de lumière (1) et le boîtier de télécommande (12) sont raccordés conjointement.

14. Dispositif selon la revendication 13, **caractérisé en ce que** l'unité de montant (14) est conçue de façon transportable et/ou coulissante.
